# FASCICULE DE BREVET EUROPEEN

(11) **EP 2 307 352 B1**
(45) Date de publication et mention de la délivrance du brevet: **19.09.2012**
(21) Numéro de dépôt: 09784499.7
(22) Date de dépôt: 09.07.2009
(51) Int. Cl.: C07C 227/08, C08G 69/08, C12P 7/40, C07C 229/08, C07C 227/04, C12P 13/00

(54) **PROCEDE DE SYNTHESE D'ACIDES OMEGA-AMINO-ALCANOÏQUES OU DE LEURS ESTERS A PARTIR D'ACIDES GRAS NATURELS**
VERFAHREN ZUR SYNTHESE VON OMEGA-AMINOALKANSÄUREN ODER DEN ESTERN DAVON AUS NATÜRLICHEN FETTSÄUREN
METHOD FOR SYNTHESISING OMEGA-AMINO-ALKANOIC ACIDS OR THE ESTERS THEREOF FROM NATURAL FATTY ACIDS

(30) Priorité: 10.07.2008 FR 0854709
(43) Date de publication de la demande: 13.04.2011
(73) Titulaire: Arkema France, 92700 Colombes (FR)
(72) Inventeur: DUBOIS, Jean-Luc, F-69390 Millery (FR)
(86) Numéro de dépôt international: PCT/FR2009/051369
(87) Numéro de publication internationale: WO 2010/004219

(56) Documents cités:
- WO-A-03/093215
- US-A- 4 474 882
- MILLER W R ET AL: "NYLON-9 VIA 9-AMINONONANOIC ACID FROM SOYBEAN OIL" IND. ENG. CHEM. RES, WEB, vol. 10, no. 4, 1 janvier 1971 (1971-01-01), pages 442-447, XP002492150 ISSN: 0536-1079
- KOHLHASE W L ET AL: "9-Aminononanamide and Nylon-9 From Azelaaldehydic Derivatives of Soybean Oil" JOURNAL OF THE AMERICAN OIL CHEMISTS' SOCIETY, SPRINGER, BERLIN, DE, vol. 47, 1 janvier 1970 (1970-01-01), pages 183-188, XP002492148 ISSN: 0003-021X
- MOL J C: "APPLICATION OF OLEFIN METATHESIS IN OLEOCHEMISTRY: AN EXAMPLE OF GREEN CHEMISTRY" GREEN CHEMISTRY, ROYAL SOCIETY OF CHEMISTRY, CAMBRIDGE, GB, vol. 4, 1 janvier 2002 (2002-01-01), pages 5-13, XP008068603 ISSN: 1463-9262

## Description

La présente invention concerne un nouveau procédé de synthèse de matériaux supramoléculaires, ainsi que les matériaux obtenus et leurs applications.

L'invention vise un procédé de synthèse d'acides ω-amino-alcanoïques ou de leurs esters à partir d'acides gras naturels mono-insaturés comportant au moins une étape de formation du diacide insaturé correspondant à l'acide gras d'origine.

L'industrie des polyamides utilise toute une gamme de monomères constitués par des ω-amino-acides à longue chaîne, usuellement appelés Nylon, caractérisés par la longueur de chaîne méthylène (-CH₂)ₙ séparant les fonctions amide -CO-NH₂-. C'est ainsi que sont connus les Nylon-6, Nylon 6-6, Nylon 6-10, Nylon 7, Nylon 8, Nylon 9, Nylon 11, Nylon 13, etc.... Ces polymères Nylon sont souvent par souci de simplification nommé PA (pour PolyAmide) en associant le nombre d'atomes de carbone de la molécule : Nylon 8 = PA-8, par exemple.

Ces monomères sont par exemple fabriqués par voie de synthèse chimique en utilisant notamment comme matière première des oléfines en C2 à C4, des cycloalcanes ou du benzène, mais aussi de l'huile de ricin (Nylon 11), de l'huile érucique ou lesquérolique (Nylon 13)...

L'évolution actuelle en matière d'environnement conduit dans les domaines de l'énergie et de la chimie à privilégier l'exploitation des matières premières naturelles provenant d'une source renouvelable. C'est la raison pour laquelle certains travaux ont été repris pour élaborer sur le plan industriel des procédés utilisant des acides/esters gras comme matière première de fabrication de ces monomères.

Ce type d'approche n'a que peu d'exemples industriels. Un des rares exemples de procédé industriel utilisant un acide gras comme matière première est celui de la fabrication à partir de l'acide ricinoléique extrait de l'huile de ricin, de l'acide amino-11-undécanoique, qui est à la base de la synthèse du Rilsan 11 ®. Ce procédé est décrit dans l'ouvrage « Les Procédés de Pétrochimie » de A. Chauvel et al. paru aux Editions TECHNIP (1986). L'acide amino-11-undécanoique est obtenu en plusieurs étapes. La première consiste en une méthanolyse de l'huile de ricin en milieu basique produisant le ricinoléate de méthyle qui est ensuite soumis à une pyrolyse pour obtenir, d'une part l'heptanaldéhyde et, d'autre part le undécylénate de méthyle. Ce dernier est passé en forme acide par hydrolyse. Ensuite l'acide formé est soumis à une hydrobromuration pour donner l'acide ω-bromé d'où l'on passe par amination à l'acide amino-11-undécanoique.

De nombreux travaux ont été conduits pour synthétiser l'acide 9-amino-nonanoïque ou acide 9-aminoazélaïque correspondant au Nylon 9 à partir de l'acide oléique d'origine naturelle.

On peut citer l'ouvrage « n-Nylons, Their Synthesis, Structure and Properties » - 1997 Ed.J. Wiley et Sons dont le chapitre 2.9 (pages 381 à 389) est consacré au 9-Nylon. Cet article donne la synthèse des réalisations et des travaux conduits sur le sujet. On y mentionne, page 384, un procédé, industriel semble-t-il, développé au Japon utilisant l'acide oléique venant l'huile de soja comme matière première. La description correspondante fait référence à l'ouvrage de A. Ravve « Organic Chemistry of Macromolecules » (1967) Marcel Dekker, Inc., dont la partie 15 est consacrée aux polyamides et qui mentionne en page 279 l'existence d'un tel procédé.

Pour être complet sur l'état de l'art en la matière, il faut citer les nombreux articles publiés par E. H. Pryde et al. entre 1962 et 1975 dans - Journal of the American Oil Chemists ' Society - « Aldehydic Materials by the Ozonization of Vegetable Oils » Vol. 39 pages 496-500 ; « Pilot Run, Plant Design and Cost Analysis for Reductive Ozonolysis of Methyl Soyate » Vol. 49 pages 643-648 et « Nylon-9 from Unsaturated Fatty Derivatives : Preparation and Characterization » Vol. 52 pages 473-477. Ces articles sont essentiellement consacrés à la réaction d'ozonolyse réductrice de l'acide gras insaturé. En effet comme il est bien connu, ainsi que cela est décrit dans l'encyclopédie Ullmann, 5 ° édition Vol. A8 pages 523 à 539 que la synthèse de diacides peut être obtenue par dégradation oxydante des acides gras insaturés, il est essentiel que la réaction d'oxydation soit conduite dans des conditions plus douces pour bloquer la réaction et parvenir à l'aldéhyde-acide CHO-R-COOH précurseur de 1'ω-amino-acide.

On peut également citer l'article de Miller W R et al. : « Nylon-9 via 9-aminononanoic acid from soybean oil » IND. ENG. CHEM. RES., WEB, Vol. 10, no. 4, 1 er janvier 1971, pages 442-447, et l'article de Kohlhase W L et al : "9-aminononanamide and Nylon-9 from azelaaldehydic derivatives of soybean oil" Journal of the American oil chemists society, Springer, Berlin, DE, vol. 47 1 er janvier 1970, pages 183-188.

L'invention vise à proposer une nouvelle voie de synthèse d'ω-amino-acide à longue chaîne à partir d'acides gras naturels provenant d'une source renouvelable.

La solution proposée consiste à travailler à partir de matières premières constituées par des acides gras mono-insaturés à longue chaîne naturels. On entend par acide gras naturel un acide issu des milieux végétal ou animal, y compris les algues, plus généralement du règne végétal, et donc renouvelable. Cet acide comportera au moins 10 et de préférence au moins 14 atomes de carbone par molécule et une insaturation oléfinique.

On peut citer à titre d'exemples de tels acides, l'acide obtusilique (4-décènoïque), l'acide caproléique (9-décénoique), l'acide lauroléique (5-dodécénoique), l'acide lindérique (4-dodécénoique), l'acide myristoléique (cis-9-tétradécénoïque), l'acide physétérique (cis-5-tétradécénoïque), l'acide tsuzuique (cis-4-tétradécénoïque), l'acide palmitoléique (cis-9-hexadécénoique), les acides en C 18, oléique (cis-9-octadécénoique), élaidique (trans-9-octadécénoique), petrosélénique (cis-6-octadécénoique), vaccénique (cis-11-octadécénoique), les acides en C 20, l'acide gadoléique (cis-9-eicosénoïque), gondoïque (cis-11-eicosénoïque) et les acides en C 22, cétoléique (cis-11-docosénoïque) et érucique (cis-13-docosénoïque), acide en C 24 acide nervonique (cis-15-tétracosénoïque).

La liste des acides gras mono-insaturés naturels avec leurs principales caractéristiques est donnée dans le tableau 1 ci-après.

**Tableau 1**

| Nom selon la Norme internationale | Nom trivial | Désignation abrégée | Masse moléculaire | Point de fusion (°C) |
|---|---|---|---|---|
| cis-4-décénoique | obtusilique | 10:1(n-6) | 170,3 | |
| cis-9-décénoique | caproléique | 10:1 (n-1) | 170,3 | |
| cis-5-lauroléique | lauroléique | 12:1(n-7) | 198,4 | |
| cis-4-dodécénoic | lindérique | 12:1(n-8) | 198,4 | |
| cis-9-tetradécénoique | myristoléique | 14:1(n-5) | 226,4 | - |
| cis-5-tétradécénoïque | physetérique | 14:1(n-9) | 226,4 | |
| cis-4-tétradécénoïque | tsuzuique | 14:1(n-10) | 226,4 | |
| cis-9-hexadécénoïque | palmitoléique | 16:1(n-7) | 254,4 | 0,5 |
| cis-6-octadécénoïque | pétrosélinique | 18:1(n-12) | 282,4 | 30 |
| cis-9-octadécénoïque | oléique | 18:1(n-9) | 282,4 | 16,2 |
| tr-9-octadécénoïque | élaidic | tr18:1(n-9) | 282,4 | 43,7 |
| cis-11-octadécénoïque | vaccénique | 18:1(n-7) | 282,4 | 39 |
| cis-9-eicosénoïque | gadoléique | 20:1(n-11) | 310,5 | 25 |
| cis-11-eicoécénoïque | gondoïque | 20:1(n-9) | 310,5 | - |
| cis-11-docosénoïque | cétoléique | 22:1(n-11) | 338,6 | |
| cis-13-docosénoïque | érucique | 22:1(n-9) | 338,6 | 33,4 |
| cis-15-tetracossénoïque | nervonique | 24: 1 (n-9) | 366,6 | 39 |

L'invention a pour objet un procédé de synthèse d'acides ω-amino-alcanoïques ou de leurs esters à partir d'acides gras naturels mono-insaturés caractérisé en ce que dans une première étape on transforme l'acide gras naturel mono-insaturé de formule générale suivante R-(CH₂)ₘ-CH=CH-(CH₂)ₚ-COOH, dans laquelle, R est soit H soit CH₃, m est un indice de valeur comprise entre 0 et 11 et p un indice de valeur comprise entre 2 et 13, en α-ω-diacide ou diester insaturé soit par réaction d'homométathèse, soit par fermentation, puis dans une deuxième étape on soumet de l'α-ω-diacide ou diester insaturé formé à une réaction de coupure oxydante pour former un seul ou deux α-ω-aldéhyde-acide ou ester différents (selon que le α-ω-diacide ou diester insaturé est symétrique ou non) de formule générale CHO-(CH₂)n-COOH , dans laquelle n est égal à m et/ou p, puis enfin à transformer le produit résultant par amination réductrice en ω-amino-acide de formule NH₂-(CH₂)ₙ₊₁-COOH

Lorsque la première étape est réalisée par homométathèse, la réaction est la suivante :

2 CH₃-(CH₂)ₘ-CH=CH-(CH₂)ₚ-COOH ⇔ COOH- (CH₂)ₚ-CH=CH- (CH₂)ₚ-COOH + CH₃- (CH₂)ₘ-CH=CH- (CH₂)ₘ-CH₃

et le diacide est séparé de l'oléfine par extraction, cristallisation, décantation ou éventuellement distillation sous vide avant d'être soumis à la deuxième étape.

Lorsque la première étape est réalisée par fermentation, l'acide est transformé en diacide : COOH-(CH₂)ₘ-CH=CH-(CH₂)ₚ-COOH et extrait du milieu de fermentation avant d'être soumis à la deuxième étape. Si dans le cas de la fermentation on obtient bien le diacide, par contre par la voie homométathèse on peut obtenir le diacide ou le diester selon le produit de départ.

Les réactions de métathèse utilisables lors de la première étape du procédé sont connues depuis longtemps même si leurs applications industrielles sont relativement limitées. On peut se référer à propos de leur utilisation dans la transformation des acides (esters) gras à l'article de J.C. Mol « Catalytic metathesis of unsaturated fatty acid esters and oil » paru dans Topics in Catalysis Vol. 27, Nos. 1-4, February 2004 (Plenum Publishing Corporation).

La catalyse de la réaction de métathèse a fait l'objet de très nombreux travaux et le développement de systèmes catalytiques sophistiqués. On peut citer par exemple les complexes au tungstène développés par Schrock et al (J. Am. Chem. Soc. 108 (1986) 2771 ou Basset et al. Angew. Chem., Ed. Engl. 31 (1992) 628. Plus récemment, sont apparus les catalyseurs dits de Grubbs ( Grubbs et al. Angew. Chem., Ed. Engl. 34 (1995) 2039 et Organic Lett. 1 (1999) 953) qui sont des complexes ruthénium-benzylidène. Il s'agit de catalyse homogène. Il a été aussi développé des catalyseurs hérétogènes à base de métaux tels que Rhénium, Molybdène et Tungstène déposés sur alumine ou silice. Enfin des travaux ont été conduits pour la réalisation de catalyseurs immobilisés, c'est-à-dire de catalyseurs dont le principe actif est celui du catalyseur homogène, notamment les complexes ruthénium-carbène, mais qui est immobilisé sur un support inactif. L'objectif de ces travaux est de mieux appréhender la sélectivité de la réaction vis-à-vis des réactions parasites entre les réactifs mis en présence. Ils portent non seulement sur la structure des catalyseurs mais également sur l'incidence du milieu réactionnel et les additifs pouvant être introduits.

Dans le procédé de l'invention tout catalyseur de métathèse actif et sélectif pourra être utilisé. On utilisera cependant de préférence des catalyseurs à base de ruthénium.

La réaction de métathèse de la première étape est conduite à une température comprise entre 20 et 100 °C et à une pression comprise entre 1 et 5 bars.

Lorsque la première étape est réalisée par fermentation on utilise un micro-organisme, tel qu'une bactérie, un champignon ou une levure permettant l'oxydation de l'acide ou ester gras de la charge. On utilisera de préférence des micro-organismes contenant des enzymes de type Oxygenase susceptibles d'oxyder la charge en formant une fonction trivalente de type acide - COOH ou ester -COOR.

Cette fermentation pourra par exemple être réalisée en présence d'une souche de *Candida tropicalis* contenant les enzymes Cytochrome P450 Monooxygenase telles que celles décrites dans la publication de W. H. Eschenfeldt et al «Transformation of fatty Acids Catalyzed by Cytochrome P450 Monooxygenase Enzymes of Candida tropicalis» paru dans Applied and Environmental Microbiology, Oct. 2003 p. 5992-5999 et les brevets FR 2,445,374 US4,474,882, US 3,823,070, US 3, 912, 586, US 6,660,505, US 6,569,670 et 5,254,466.

La deuxième étape du procédé consiste en une coupure oxydante de la double liaison du diacide.

La réaction de coupure oxydante de la double liaison, qui conduit à la formation de fonctions aldéhydes sur les deux carbones de la double liaison, est également en elle-même connue. Elle peut être réalisée au moyen d'un oxydant fort tel que KMnO₄ sous forme concentrée et à la chaleur ainsi que cela est décrit dans « Organic Chemistry » de L.G. Wade Jr. 5th Edition Chapter 8 Reactions of Alkenes. La coupure oxydante peut être effectuée avec de l'eau oxygénée comme décrit dans le brevet GB 743491. L'article de F. Drawert et al. dans Chem. Mikrobiol. Technol. Lebensm. 1, 158-159 (1972) décrit une voie alternative par irradiation de l'huile de tournesol. D'autre part l'article de G.S. Zhang et al. dans Chinese Chemical Letters, Vol 5, N°2, pp105-108 de 1994, indique qu'il est possible d'effectuer la coupure oxydante à partir du diol correspondant à l'acide oléique (voir Entrée 29 du tableau). Cette coupure oxydante est effectuée en utilisant le Chlorochromate d'ammonium comme oxydant. Le diol quant à lui est obtenu par époxydation de l'acide oléique suivie d'une hydrolyse du pont époxy. Elle peut être réalisée par d'autres oxydants tels que l'eau oxygénée et plus particulièrement l'ozone.

Il faut cependant éviter que la réaction d'oxydation soit complète car, comme cela a été indiqué ci-dessus, l'oxydation d'un acide insaturé est la voie de synthèse bien connue pour la fabrication des diacides. Il y a donc lieu de prévoir des conditions opératoires telles que l'on s'arrête à la fonction aldéhyde. C'est la raison pour laquelle lors des travaux décrits dans l'art antérieur on s'est intéressé à une réaction d'oxydation couplée avec une réduction, souvent l'hydrogénation, des produits de l'oxydation, très généralement obtenue par ozonolyse. Les conditions d'oxydation doivent donc être conduites dans des conditions plus douces pour ainsi mieux maîtriser le processus en travaillant en présence d'hydrogène associé à un catalyseur et/ou d'un agent réducteur doux. C'est cette réaction que l'on dénomme l'ozonolyse réductrice.

La réaction d'ozonolyse a fait l'objet de travaux importants qui ont permis de dégager un mécanisme réactionnel dit de « Criegee » (cf Article « Aldehydic Materials by the Ozonization of Vegetable Oils » Vol. 39 pages 496-500 , cité ci-dessus) marqué par la formation d'un ozonide.

La première phase de l'ozonolyse réductrice peut être réalisée dans différents milieux solvants. Si elle est conduite en phase aqueuse, l'acide gras insaturé est présent sous la forme d'une émulsion eau dans l'huile. Elle peut être conduite dans un solvant de type alcool, méthanol, éthanol, propanol, butanol, méthoxyéthanol, cyclohexanol, alcool benzylique ; dans le cas où l'ozonolyse est réalisée sur l'ester gras, il sera avantageux d'utiliser l'alcool R-OH correspondant à l'ester gras. Il a également été proposé par Chris Schwartz, Joseph Raible, Kyle Mott, et Patrick H. Dussault, Tetrahedron 62 (2006), pp. 10747-10752, d'utiliser le DMSO comme milieu solvant. Au milieu solvant alcool, il est souvent d'associer un acide organique, en général, l'acide acétique qui sera présent en général sous forme d'un mélange équimoléculaire avec l'alcool.

La seconde phase de l'ozonolyse réductrice va consister en une réduction de l'ozonide qui peut être réalisée avec du zinc dans l'acide acétique, une hydrogénation en présence d'un catalyseur d'hydrogénation (Pd par exemple) ou à l'aide d'un agent réducteur tel que par exemple le diméthylesulfure (DMS).

La variante préférée de réalisation de cette étape est l'ozonolyse réductrice qui pourra réalisée en présence de zinc métal, sous forme de poudre, ou encore de préférence en présence de diméthylesulfure (DMS :CH3-S-CH3); en effet ce DMS sera transformé lors de l'ozonolyse réductrice en DMSO, solvant largement utilisé par l'industrie.

Enfin, l'amination réductrice de la fonction aldéhyde en amine primaire est bien connue de l'homme de l'art. L'amination réductrice de l'acide 9-oxononanoïque obtenu pour former l'acide amino-9-nonanoïque peut être effectuée selon de nombreuses méthodes, catalytiques ou enzymatiques, et par exemple selon la méthode décrite dans la demande de brevet US 5,973,208.

Les mécanismes réactionnels du procédé, dans ses deux versions, peuvent être résumés comme suit.
- 1^{ère} étape : homométathèse : formation du diacide gras

   2 CH₃-(CH₂)ₘ-CH=CH-(CH₂)ₚ-COOH ⇔ COOH-(CH₂)ₚ-CH=CH-(CH₂)ₚ-COOH + CH₃-(CH₂)ₘ-CH=CH- (CH₂)ₘ-CH₃

   suivie éventuellement d'une estérification après elimination de l'oléfine
- 2^{ème} étape : après élimination de l'oléfine: coupure oxydante (Ozonolyse réductrice)

   COOH-(CH₂)ₚ-CH=CH-(CH₂)ₚ-COOH (Oxydant, H₂*) → 2 CHO- (CH₂)ₚ-COOH + H₂O

   H₂* symbolyse dans la réaction 3, le couplage d'une oxydation suivie d'une réduction.
- 3^{éme} étape : Amination réductrice

   2 CHO-(CH₂)ₚ-COOH + (NH₃, H₂) → 2 NH₂-(CH₂)ₚ₊₁-COOH + 2 H₂O
et dans le cas de la fermentation,
- 1^{ére} étape : fermentation : formation du diacide gras

   CH₃-(CH₂)ₘ-CH=CH-(CH₂)ₚ-COOH → COOH-(CH₂)ₘ-CH=CH- (CH₂)ₚ-COOH

   suivie éventuellement d'une estérification
- 2^{ème} étape : après extraction du diacide : coupure oxydante (Ozonolyse réductrice)

   COOH-(CH₂)ₘ-CH=CH-(CH₂)ₚ-COOH (Oxydant, H₂*) → CHO-(CH₂)ₘ-COOH + CHO-(CH₂)ₚ-COOH + H₂O
- 3^{ème} étape : Amination réductrice

   CHO-(CH₂)ₘ-COOH + CHO-(CH₂)ₚ-COOH + (NH₃, H₂) → NH₂-(CH₂)ₘ₊₁-COOH + NH₂-(CH₂)ₚ₊₁-COOH + 2 H₂O

Lorsque la première étape de fermentation ou d'homométathèse/élimination de l'oléfine est suivie d'une estérification puis des étapes de coupure oxydante et d'amination réductrice, on obtient un ester-amine qui peut être distillé et polymérisé directement (production de méthanol) ou hydrolysé en amino-acide et ensuite polymérisé.

C'est ainsi qu'en appliquant ces mécanismes réactionnels aux divers acides gras cités dans le Tableau 1 on est susceptible de produire les produits suivants.

### PA 9 par la voie homométathèse de l'Acide caproléique

L'acide lauroléique conduit par la voie homométathèse à du PA 5, et par la voie fermentation à un mélange de PA 5 et de PA 7 qui ne présentent pas de propriétés spécialement intéressantes dans l'application polymérisation.

L'acide lindérique conduit par la voie homométathèse à du PA 4 et par la voie fermentation à un mélange de PA 4 et de PA 8 peu intéressant.

L'acide myristoléique conduit par la voie homométathèse à du PA 9 et par la voie fermentation un mélange de PA 9 et de PA 5.

L'acide physitérique conduit par la voie homométathèse à du PA 5, par la voie fermentation un mélange de PA 5 et de PA 9.

L'acide tsuzuique conduit par la voie homométathèse à du PA 4 et par la voie fermentation à un mélange de PA 4 et de PA 10.

L'acide palmitoléique conduit par la voie homométathèse à du PA 9 et par la voie fermentation à un mélange de PA 9 et de PA 7.

L'acide pétrosélénique conduit par la voie homométathèse à du PA 6 et par la voie fermentation à un mélange de PA 6 et de PA 12.

Les acides oléiques conduisent par les 2 voies à du PA 9 avec double rendement car c'est le seul produit qui peut être obtenu.

L'acide vaccénique conduit par la voie homométathèse à du PA 11 et par la voie fermentation un mélange de PA 11 et de PA 7.

L'acide gadoléique conduit par la voie homométathèse à du PA 9 et par la voie fermentation à un mélange de PA 9 et de PA 11.

L'acide gondoïque conduit par la voie homométathèse à du PA 11 et par la voie fermentation à un mélange de PA 9 et de PA 11

L'acide cétoléique conduit par les 2 voies à du PA 11 avec double rendement.

L'acide érucique conduit: par la voie homométathèse à du PA 13 et par la voie fermentation à un mélange de PA 13 et de PA 9.

L'acide nervonique conduit par voie homométathèse à du PA15 et par fermentation à un mélange de PA15 et PA9.

Ainsi qu'on peut l'observer le procédé appliqué à certains acides gras naturels, les acides gras insaturés symétriques (m = p) permet d'obtenir des performances, rendements assez exceptionnels dans la mesure où avec une seule molécule d'acide gras on obtient 2 molécules d'ω-aminoacides. Il s'agit des acides amino-9-nonanoïque (PA-9) issus des acides en C-18 oléiques et amino-11 - undécanoïque (PA-11) issu de l'acide cétoléique qui constituent des monomères de polymérisation particulièrement intéressants.

Naturellement, le critère économique, à côté des performances techniques, joue un rôle prépondérant. Certains de ces acides sont largement disponibles ce qui leur procurent un avantage indéniable dès lors qu'ils sont susceptibles de conduire à un monomère industrialisables.

Parmi ces acides on peut citer l'acide caproléique, l'acide myristoléique, l'acide palmitoléique et les acides oléiques qui mènent tous au PA-9

On peut observer également que le procédé mis en oeuvre dans sa variante fermentation permet avec certains autres acides gras naturels d'obtenir des mélanges d'ω-aminoacides de structures voisines et/ ou complémentaires, mélanges qui sont susceptibles d'être polymérisé en conduisant à des performances analogues à celles obtenues avec des monomères purs.

On peut citer à ce sujet :
l'acide myristoléique qui conduit à un mélange PA-5 et PA-9,
l'acide palmitoléique qui conduit à un mélange PA-7 et PA-9,
l'acide vaccénique qui conduit à un mélange PA-7 et PA-11
l'acide gadoléique qui conduit à un mélange PA-9 et PA-11,
l'acide gondoléique qui conduit à un mélange PA-9 et PA-11, et
l'acide érucique qui conduit à un mélange de PA 9 et de PA 13.

Le procédé de l'invention est illustré par les exemples ci-après

### Exemple 1 : Voie Fermentation appliquée à l'acide oléique.

### 1^{ère} étape

Dans cet exemple on utilisera une levure contenant au moins une enzyme Oxygénase. La levure sera cultivée à pH=7, dans un milieu d'eau désionisée contenant du sorbitol, des oligoéléments, de l'urée et de l'acide oléique. Le mélange sera ensuite stérilisé à 120 °C pendant 15 minutes. Une souche de levure sera ensuite inoculée au milieu de culture. La culture sera maintenue à 30 °C. Une solution de soude sera ajoutée en continue pour maintenir le milieu à un pH de 7.0 à 7.5. Après 48 heures de culture, le diacide insaturé sera récupéré par extraction au diéthyléther. Après élimination du solvant par évaporation, on récupèrera des cristaux qui après recristallisation auront un point de fusion de 69 °C, c'est à dire équivalent à celui décrit pour le diacide 9-octadécènedioïque.

### 2^{ème} étape

Le diacide 9-octadécènedioïque de première étape sera mis en solution dans du pentane saturé d'ozone et soumis à une ozonolyse réductrice. Cet exemple illustre la coupure oxydante du diacide C18 issu de l'exemple 1 par ozonolyse réductrice.

1 mg du diester de l'acide oléique : diméthyl -1,18 octadéc-9-énoate, est dissout dans 2 ml de pentane saturé d'ozone et prérefroidis à -70 °C. Le pentane est évaporé sous flux d'azote et 1 ml de DMS est ajouté à l'ozonide obtenu. Après 30 minutes, l'excès de DMS est évaporé sous flux d'azote. Le produit est dissout dans une petite quantité d'éther et est analysé.

Le rendement en ester 9-oxononanoate de méthyle est de 82 % molaire.

### 3ème étape

Le composé issu de la deuxième étape de formule CHO-(CH2)7-COOH acide 9-oxononanoique sera soumis à une amination réductrice dans les conditions suivantes.

Dans un autoclave en acier inoxydable de 500 ml, on verse 50 g d'aldéhyde ester, 50 ml d'ammoniac liquide, 125 ml d'alcool et 6 g de Nickel de Raney.

L'hydrogène est introduit à une pression de 100 à 150 atmosphères et l'autoclave est chauffé à 100 - 110 °C pendant 4 heures. Au refroidissement l'hydrogène et l'ammoniac sont chassés, le contenu est siphonné et l'autoclave est rincé avec l'alcool. Le contenu de l'autoclave et l'alcool de lavage sont rassemblés, essorés sur Büchner et placés dans un appareil à distiller sous vide en présence d'azote. L'alcool et l'ammoniac sont chassés à la trompe à eau puis à la pompe à palettes. L'aminoester brut, coloré, est placé dans une ampoule à brome en vue de sa distillation dans l'appareil décrit.

L'aminoester distillé (38 g) est légèrement coloré. Le rendement est de 76 % molaire.

L'aminoester peut éventuellement directement polymérisé en polyamide-9, par chauffage sous vide pour récupérer le méthanol produit.

On peut aussi polymériser l'aminoacide. Pour cela on procède à une hydrolyse de l'amino ester. L'amino-9-nonanoate de méthyle obtenu à partir de 28 g d'aldéhyde ester est placé dans une ampoule à brome pour tomber goutte à goutte dans un ballon tricol de 2 litres surmonté d'une longue colonne à distiller et contenant un litre d'eau bouillante. Le reflux est réglé de façon à distiller le méthanol formé, ce qui permet de suivre la réaction, l'hydrolyse dure 4 à 5 heures pour l'ester méthylique. Lorsque la réaction est terminée, on filtre à chaud et évapore l'eau. On obtient un produit difficile à sécher au dessiccateur alors qu'en lavant le produit humide à l'acétone et en le séchant au dessiccateur, on recueille 20 g d'acide aminé brut incolore.

### Exemple 2 : Voie Homométathèse appliquée à l'acide oléique

### 1ère étape

Cet exemple illustre la deuxième étape (facultative) d'homométathèse de l'acide oléique en diacide de formule COOH- (CH₂)₇- CH=CH-(CH₂)₇-COOH.

Pour cette deuxième étape, on utilise le catalyseur complexe bispyridine ruthénium (8) décrit dans la publication de Chen-Xi Bai et al., Tetrahedron Letters, 46 (2005) 7225-7228. La réaction est effectuée dans le toluène, à une température de 50°C et pendant 12 heures sous une pression de 100 kPa en extrayant en cours de réaction l'éthylène formée. Le rendement en diacide 9-octadécènedioïque est de 85 % molaire.

Ce produit sera soumis au traitement des deuxième et troisième étapes décrites ci-dessus et on obtiendra l'acide amino-11 undecénoique.

## Revendications

1. Procédé de synthèse d'acides ω-amino-alcanoïques ou de leurs esters à partir d'acides gras naturels mono-insaturés **caractérisé en ce que** dans une première étape on transforme l'acide gras naturel mono-insaturé de formule générale suivante R-(CH₂)ₘ-CH=CH-(CH₂)ₚ-COOH, dans laquelle, R est soit H soit CH₃, m est un indice de valeur comprise entre 0 et 11 et un p un indice de valeur comprise entre 2 et 13, en α-ω-diacide ou diester insaturé soit par réaction d'homométathèse, soit par fermentation, puis dans une deuxième étape on soumet de l'α-ω-diacide ou diester insaturé formé à une réaction de coupure oxydante pour former un seul ou deux α-ω-aldéhyde-acide ou ester différents de formule générale CHO-(CH₂)ₙ-COOH , dans laquelle n est égal à m et/ou p, puis enfin à transformer le produit résultant par amination réductrice en ω-amino-acide de formule NH₂-(CH₂)ₙ₊₁-COOH.

2. Procédé selon la revendication 1 **caractérisé en ce que** le diacide issu de la première étape est soumis à une ozonolyse dans un milieu solvant et des conditions réductrices.

3. Procédé selon la revendication 2 **caractérisé en ce que** les conditions réductrices sont apportées par de l'hydrogène associé à du zinc métal.

4. Procédé selon la revendication 2 **caractérisé en ce que** les conditions réductrices sont apportées par du diméthylsulfure.

5. Procédé selon l'une des revendications 1 à 4 **caractérisé en ce que** la première étape est réalisée par homométathèse de l'acide gras

6. Procédé selon la revendication 5 **caractérisé en ce que** la réaction de métathèse est conduite à une température comprise entre 20 et 100 °C et à une pression comprise entre 1 et 5 bars en présence de catalyseurs à base de ruthénium.

7. Procédé selon l'une des revendications 1 à 4 **caractérisé en ce que** la première étape est réalisée par fermentation de l'acide gras.

8. Procédé selon la revendication 7 **caractérisé en ce que** la fermentation sera réalisée en présence de micro-organismes contenant des enzymes de type Oxygenase susceptibles d'oxyder la charge en formant une fonction trivalente de type acide -COOH ou ester -COOR.

9. Procédé selon la revendication 1 **caractérisé en ce que** le diacide obtenu à l'issue de la première étape est soumis à une estérification.

## Claims

1. Process for synthesizing ω-aminoalkanoic acids, or esters thereof, from monounsaturated natural fatty acids, **characterized in that**, in a first step, the monounsaturated natural fatty acid having the following general formula R- (CH₂)ₘ-CH=CH- (CH₂)ₚ-COOH, in which R is either H or CH₃, m is an index having a value of between 0 and 11 and p is an index having a value of between 2 and 13, is converted into an unsaturated α-ω-diacid or diester, either by a homometathesis reaction or by fermentation, then, in a second step, the unsaturated α-ω-diacid or diester formed is subjected to an oxidative cleavage reaction so as to form a single α-ω-aldehyde-acid or ester or two different α-ω-aldehyde-acids or esters of general formula CHO-(CH₂)ₙ-COOH, in which n is equal to m and/or p, and then, finally, the resulting product is converted, by reductive amination, into an ω-amino acid of formula NH₂-(CH₂)ₙ₊₁-COOH.

2. Process according to Claim 1, **characterized in that** the diacid resulting from the first step is subjected to ozonolysis in a solvent medium and reducing conditions.

3. Process according to Claim 2, **characterized in that** the reducing conditions are provided by hydrogen combined with zinc metal.

4. Process according to Claim 2, **characterized in that** the reducing conditions are provided by dimethyl sulfide.

5. Process according to one of Claims 1 to 4, **characterized in that** the first step is carried out by homometathesis of the fatty acid.

6. Process according to Claim 5, **characterized in that** the metathesis reaction is carried out at a temperature of between 20 and 100°C and at a pressure of between 1 and 5 bar, in the presence of ruthenium-based catalysts.

7. Process according to one of Claims 1 to 4, **characterized in that** the first step is carried out by fermentation of the fatty acid.

8. Process according to Claim 7, **characterized in that** the fermentation will be carried out in the presence of microorganisms containing enzymes of oxygenase type which are capable of oxidizing the feedstock, forming a trivalent function of acid - COOH or ester -COOR type.

9. Process according to Claim 1, **characterized in that** the diacid obtained at the end of the first step is subjected to an esterification.

## Patentansprüche

1. Verfahren zur Synthese von ω-Aminoalkansäuren oder Estern davon aus einfach ungesättigten natürlichen Fettsäuren, **dadurch gekennzeichnet, dass** man in einem ersten Schritt die einfach ungesättigte natürliche Fettsäure der folgenden allgemeinen Formel R-(CH₂)ₘ-CH=CH-(CH₂)ₚ-COOH, worin R entweder für H oder für CH₃ steht, m für einen Index mit einem Wert zwischen 0 und 11 steht und p für einen Index mit einem Wert zwischen 2 und 13 steht, entweder durch Homometathese oder durch Fermentation in eine ungesättigte α,ω-Disäure bzw. einen ungesättigten α,ω-Disäurediester umwandelt, dann in einem zweiten Schritt die gebildete ungesättigte α,ω-Disäure bzw. den gebildeten ungesättigten α,ω-Disäurediester einer Oxidationsspaltungsreaktion unterwirft, wobei man eine einzige α,ω-Aldehydsäure bzw. einen einzigen α,ω-Aldehydsäureester oder zwei verschiedene α,ω-Aldehydsäuren bzw. α,ω-Aldehydsäureester der allgemeinen Formel CHO-(CH₂)ₙ-COOH, worin n gleich m und/oder p ist, erhält, und dann das resultierende Produkt durch reduktive Aminierung in eine ω-Aminosäure der Formel NH₂-(CH₂)ₙ₊₁-COOH umwandelt.

2. Verfahren nach Anspruch 1, **dadurch gekennzeichnet, dass** man die Disäure aus dem ersten Schritt einer Ozonolyse in einem Lösungsmittelmedium und reduzierenden Bedingungen unterwirft.

3. Verfahren nach Anspruch 2, **dadurch gekennzeichnet, dass** man die reduzierenden Bedingungen durch Wasserstoff in Kombination mit Zinkmetall herbeiführt.

4. Verfahren nach Anspruch 2, **dadurch gekennzeichnet, dass** man die reduzierenden Bedingungen durch Dimethylsulfid herbeiführt.

5. Verfahren nach einem der Ansprüche 1 bis 4, **dadurch gekennzeichnet, dass** man den ersten Schritt durch Homometathese der Fettsäure durchführt.

6. Verfahren nach Anspruch 5, **dadurch gekennzeichnet, dass** man die Metathesereaktion bei einer Temperatur zwischen 20 und 100°C und einem Druck zwischen 1 und 5 bar in Gegenwart von Katalysatoren auf Basis von Ruthenium durchführt.

7. Verfahren nach einem der Ansprüche 1 bis 4, **dadurch gekennzeichnet, dass** man den ersten Schritt durch Fermentation der Fettsäure durchführt.

8. Verfahren nach Anspruch 7, **dadurch gekennzeichnet, dass** man die Fermentation in Gegenwart von Mikroorganismen, die Enzyme vom Oxygenase-Typ, die zur Oxidation des Einsatzstoffs unter Bildung einer dreiwertigen Funktion vom -COOH-Säure-Typ oder -COOR-Ester-Typ befähigt sind, enthalten, durchführt.

9. Verfahren nach Anspruch 1, **dadurch gekennzeichnet, dass** man die Disäure aus dem ersten Schritt einer Veresterung unterwirft.
